# EUROPEAN PATENT APPLICATION

(11) **EP 3 747 468 A1**
(43) Date of publication of application: **09.12.2020**
(21) Application number: 19743299.0
(22) Date of filing: 29.01.2019
(51) Int. Cl.: A61K 45/00, A61K 31/7088, A61P 25/28, A61P 43/00, G01N 33/15, G01N 33/50

(54) **THERAPEUTIC AGENT FOR FRONTOTEMPORAL LOBAR DEGENERATION, METHOD FOR SCREENING THERAPEUTIC AGENT FOR FRONTOTEMPORAL LOBAR DEGENERATION AND METHOD FOR TREATING FRONTOTEMPORAL LOBAR DEGENERATION**

(30) Priority: 29.01.2018 US 201862623203 P
(71) Applicant: National University Corporation Tokyo Medical and Dental University, Tokyo 113-8510 (JP)
(72) Inventor: OKAZAWA Hitoshi, Tokyo 113-8510 (JP)
(74) Representative: Berggren Oy, Helsinki & Oulu
(86) International application number: PCT/JP2019/003018
(87) International publication number: WO 2019/146805

(57) **Abstract**

An object of the present invention is to provide a novel therapeutic agent for frontotemporal lobar degeneration. The present invention provides a therapeutic agent for frontotemporal lobar degeneration containing an active ingredient which suppresses gene expression or protein function of Gas6 and/or Tyro3. The active ingredient may contain a nucleic acid.

SEQUENCE LISTING

<110>  National University Corporation Tokyo Medical and Dental University

<120>  THERAPEUTIC AGENT FOR FRONTOTEMPORAL LOBAR DEGENERATION, SCREENING
METHOD AND TREATMENT METHOD

<130>  P17-032P

<160>  10

<170>  PatentIn version 3.5

<210>  1
<211>  22
<212>  DNA
<213>  Artificial Sequence

<220>
<223>  Artificial Sequence

<400>  1
cgtgcaatcc atcttgttca at                                                22


<210>  2
<211>  25
<212>  DNA
<213>  Artificial Sequence

<220>
<223>  Artificial Sequence

<400>  2
catgacctaa ctcaatgcat accac                                             25


<210>  3
<211>  25
<212>  DNA
<213>  Artificial Sequence

<220>
<223>  Artificial Sequence

<400>  3
ctgtgtctca ctagaagcat aagca                                             25


<210>  4
<211>  25
<212>  DNA
<213>  Artificial Sequence

<220>
<223>  Artificial Sequence

<400>  4
actagattgg gaaagacagt gaatc                                             25


<210>  5
<211>  24

<212>  DNA
<213>  Artificial Sequence

<220>
<223>  Artificial Sequence

<400>  5
aattccaatc ctgtgtggtc atag                                              24


<210>  6
<211>  24
<212>  DNA
<213>  Artificial Sequence

<220>
<223>  Artificial Sequence

<400>  6
ccactttctt tctcccttac ccta                                              24


<210>  7
<211>  30
<212>  DNA
<213>  Artificial Sequence

<220>
<223>  Artificial Sequence

<400>  7
gaacaagatg gattgcacgc aggttctccg                                        30


<210>  8
<211>  24
<212>  DNA
<213>  Artificial Sequence

<220>
<223>  Artificial Sequence

<400>  8
gtagccaacg ctatgtcctg atag                                              24


<210>  9
<211>  25
<212>  DNA
<213>  Artificial Sequence

<220>
<223>  Artificial Sequence

<400>  9
gatctttcta tgtacagcca cgttt                                             25


<210>  10
<211>  25
<212>  DNA
<213>  Artificial Sequence

<220>
<223>  Artificial Sequence

<400>  10
ctaatggtgt tcaacgtcaa gtagt                                          25

## Description

### TECHNICAL FIELD

The present invention relates to a therapeutic agent for frontotemporal lobar degeneration, a method for screening a therapeutic agent for frontotemporal lobar degeneration, and a method for treating frontotemporal lobar degeneration.

### BACKGROUND ART

Frontotemporal lobar degeneration, Alzheimer's disease, and Lewy body dementia are known as three major dementias. However, no radical treatment has been established for these three major dementias; in particular, therapies of frontotemporal lobar degeneration tend to be under delayed development.

Each of the three major dementias includes familial dementia, which is strongly affected by inheritance, and sporadic dementia, in which the genetic factors are not pronounced. In familial frontotemporal lobar degeneration, it has been reported that the frequency of genetic mutations of tau and progranulin (PGRN) is relatively high (e.g., Non-Patent Document 1). It has also been reported that the phosphorylation of the MAPK signaling pathway plays a key role in the pathogenic mechanism of frontotemporal lobar degeneration (Patent Document 1).

Patent Document 1: PCT International Publication No. WO2015/099094

Non-Patent Document 1: Scientific Reports volume 7, Article number: 1513 (2017)

### DISCLOSURE OF THE INVENTION

### Problems to be Solved by the Invention

However, there exists an additional need for the identification of target molecules capable of ameliorating clinical symptoms in the treatment of frontotemporal lobar degeneration.

The present invention was made in view of the foregoing circumstances, and an object of the present invention is to provide a novel therapeutic agent for frontotemporal lobar degeneration.

### Means for Solving the Problems

The present inventors have found that in frontotemporal lobar degeneration, abnormal phosphorylation takes place at a specific site in the tau protein and consequently the abnormally phosphorylated tau protein impairs synapses. Further, the present inventors have elucidated the upstream of the abnormal phosphorylation, and found Gas6 and Tyro3 as target molecules for frontotemporal lobar degeneration. Based on these findings, the present inventors have completed the present invention. More specifically, the present invention provides the following.

A first aspect of the present invention relates to a therapeutic agent for frontotemporal lobar degeneration, containing an active ingredient which suppresses the gene expression or protein function of Gas6 and/or Tyro3.

A second aspect of the present invention relates to the therapeutic agent for frontotemporal lobar degeneration according to the first aspect, in which the active ingredient contains a nucleic acid.

A third aspect of the present invention relates to a method for screening a therapeutic agent for frontotemporal lobar degeneration, including administering a candidate compound to a disease model mouse of frontotemporal lobar degeneration, in which the progranulin gene of the disease model mouse is modified by knock-in, and screening the candidate compound based on the phosphorylation of Ser203 in tau protein as an indicator.

A fourth aspect of the present invention relates to a method for treating frontotemporal lobar degeneration, including administering to a patient the therapeutic agent for frontotemporal lobar degeneration according to the first or second aspect.

### Effects of the Invention

The present invention makes it possible to provide a novel therapeutic agent for frontotemporal lobar degeneration.

### BRIEF DESCRIPTION OF THE DRAWINGS

FIG. 1 is a schematic diagram of a pathogenic mechanism of FTLD-TDP;
FIG. 2 is a diagram showing the results of the morphological analysis of spines for the case of the knockdown of the tau gene;
FIG. 3 is a diagram showing the results of the morphological analysis of spines for the case of the knockdown of the BRAF gene;
FIG. 4 is a diagram showing the results of the morphological analysis of spines for the case of the knockdown of the PKCα gene;
FIG. 5 is a diagram showing the results of the morphological analysis of spines for the case of the knockdown of the Tyro3 gene;
FIG. 6 is a diagram showing the results of the morphological analysis of spines for the case of the knockdown of the Gas6 gene;
FIG. 7 is a diagram showing the results of the morphological analysis of spines for the case of the administration of a BRAF inhibitor; and
FIG. 8 is a diagram showing the results of the morphological analysis of spines for the case of the administration of a PKC inhibitor.

### PREFERRED MODE FOR CARRYING OUT THE INVENTION

Hereinafter, embodiments of the present invention will be described, but the present invention is not limited thereto.

### <Therapeutic Agent for Frontotemporal Lobar Degeneration>

A therapeutic agent for frontotemporal lobar degeneration according to an embodiment of the present invention (hereinafter, also referred to as "therapeutic agent of the present invention") contains an active ingredient which suppresses the gene expression or protein function of Gas6 (growth-arrest specific gene 6) and/or Tyro3 (one of receptor tyrosine kinases).

In the present invention, "frontotemporal lobar degeneration (FTLD)" means a non-Alzheimer's disease type neurodegenerative disease which exhibits atrophy in the frontal and temporal lobes in the early stage, and leads to atrophy of the entire brain in the late stage. Frontotemporal lobar degeneration includes three types of diseases classified according to their clinical features, i.e., frontotemporal dementia (FTD), progressive non-fluent aphasia (PNFA) and semantic dementia (SD). In addition, frontotemporal lobar degeneration includes four types of diseases pathologically classified according to the type of the protein accumulated in cells as an abnormal protein, i.e., FTLD-Tau, FTLD-TDP, FTLD-UPS and FTLD-FUS.

"FTLD-Tau" is classified into "3R Tau" type, "4R Tau" type and "3/4R Tau" type according to the number of microtubule binding domains repeated in Tau protein which is predominantly accumulated in the cells. Moreover, the "3R Tau" type includes FTLD with Pick bodies (Pick's disease), FTLD with MAPT (microtubule-associated protein Tau) gene mutation (FTLD-17), and the like. The "4R Tau" type includes corticobasal degeneration, progressive supranuclear palsy, multiple system tauopathy with dementia, argyrophilic grain dementia (argyrophilic grain disease), FTLD with MAPT gene mutation (FTLD-17), and the like. The "3/4R Tau" type includes dementia with neurofibrillary tangles, FTLD with MAPT gene mutation (FTLD-17), and the like.

A group of FTLDs having tau-negative, ubiquitin-positive inclusions is called "FTLD-U", and includes FTLD-TDP, FTLD-UPS and FTLD-FUS described above.

"FTLD-TDP" means a TDP-43-positive disease, among FTLD-Us. This disease includes FTLD with PGRN (progranulin gene) mutation, FTLD with sporadic FTLD-TDP/FTLD-U, FTLD with TARDBP (TDP-43 gene) mutation, FTLD with VCP (valosin-containing protein gene) mutation, FTLD linked to chromosome 9, and the like.

"FTLD-UPS" is one of FTLD-Us which is TDP-43-negative. The disease includes FTLD with CHMP2B (charged polyvacuolar protein 2B gene) mutation, and the like.

"FTLD-FUS" means, among FTLD-Us, a disease that is TDP-43-negative and FUS (fused in sarcoma)-positive. The disease includes neuronal intermediate filament inclusion disease, atypical FTLD-U, basophilic inclusion body disease, FTLD with FUS mutation, and the like.

"Developing frontotemporal lobar degeneration" and related phrases mean the expression of symptoms such as memory disorder, higher brain function disorders (aphasia, apraxia, agnosia, constructional apraxia), and change in personality as determined by clinical diagnosis, as well as the appearance of atrophy in the brain as determined by diagnostic imaging. "Affected with frontotemporal lobar degeneration" and related phrases mean to also include a condition in which the aforementioned symptoms are not expressed but a pathological change peculiar to frontotemporal lobar degeneration (e.g., accumulation of the abnormal protein in cells) has occurred.

A subject to be treated with the therapeutic agent of the present invention includes both patients who have developed frontotemporal lobar degeneration and patients affected with from frontotemporal lobar degeneration.

In the present invention, "treatment" means, for example, delaying the progression of a symptom of frontotemporal lobar degeneration as well as mitigating, alleviating, ameliorating or healing the symptom, or the like.

The present invention is particularly effective for the treatment of FTLD-TDP among the types of frontotemporal lobar degeneration.

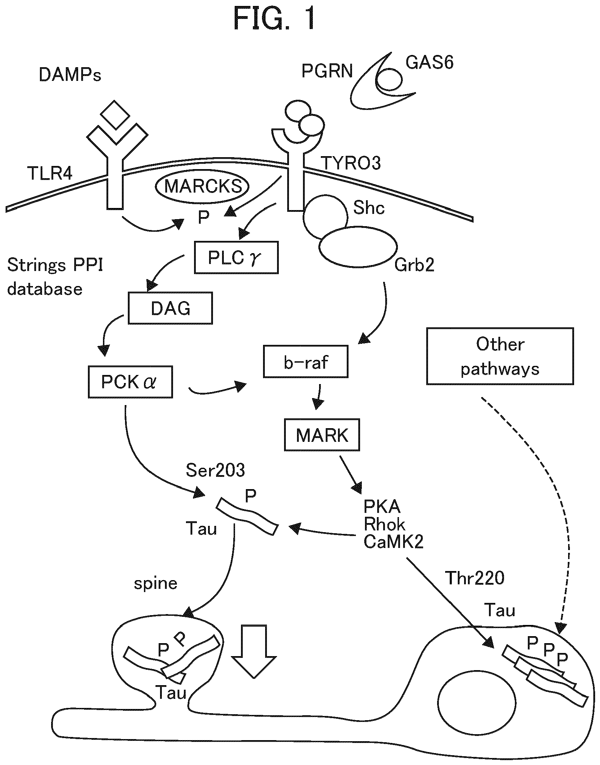

Among the types of frontotemporal lobar degeneration, the present inventor has elucidated the pathology of FTLD-TDP in which an aggregated protein of TDP43 (TAR DNA-binding protein of 43 kDa) is found. The summary of a mechanism that has been consequently identified is outlined in FIG. 1.

It is known that the aggregated protein of TDP43 can be caused by a mutation in a gene such as progranulin, VCP (valosin-containing protein), TDP43, C9orf72 (Chromosome 9 open reading frame 72), and the like. Based on this, the present inventors prepared "PGRN-KI mice" which are disease model mice of frontotemporal lobar degeneration (for details of the preparation method, etc., see Examples, or WO2015/099094). The mice are progranulin-knock-in mice.

"Progranulin (PGRN)" is a protein of 88 kD and is thought to have anti-inflammatory and neurotrophic effects. "Knock-in" means a genetic engineering procedure in which a complementary DNA sequence encoding a protein is inserted into a specific locus in an organism's chromosome. Knock-in mice, which are mice prepared using this procedure, are considered to be more natural mouse models because, unlike transgenic mice, they do not cause an increase in the number of genes of interest (copy number) or an excessive amount of gene expression, which would be caused by an artificial gene regulatory region (enhancer, and/or promoter).

The present inventor performed a comprehensive phosphoproteomic analysis over time using cerebral cortical tissues from the PGRN-KI mice. As a result, abnormally increased phosphorylation of the 203rd amino acid (Ser203) of the tau protein was unexpectedly detected in synapses of neurons prior to intracerebral aggregation of the TDP43 protein. A decrease in the number of synapses was also found.

The Ser203-phosphorylated mouse tau protein (pSer203 mouse tau protein) corresponds, in humans, to human tau protein which is phosphorylated at the 214th amino acid (Ser214) in the tau protein (pSer214 human tau protein). As a result of the investigation performed by the present inventors, the pSer214 human tau protein was also found in the synapses of the brain neurons of human frontotemporal lobar degeneration patients. Such localization of the abnormally phosphorylated tau proteins in the synapses has also been reported in models of Alzheimer's disease, suggesting that frontotemporal lobar degeneration and Alzheimer's disease are caused by common pathologies.

It is extremely likely that the localization of the tau protein phosphorylated at the site corresponding to Ser203 of the mouse tau protein in the synapses leads to frontotemporal lobar degeneration, even in animals other than the mice and human. It is to be noted that in various animals other than the mice, the site corresponding to Ser203 of the mouse tau protein can be easily identified based on the tau protein sequence of each animal.

Based on the above findings, the expression of the tau protein in the PGRN-KI mice was suppressed using an adeno-associated viral (AAV) knockdown vector of the tau protein. This suppression normalized the number of synapses that had been reduced, and ameliorated the symptoms of the cognitive impairment. Therefore, it was found that reduced expression of progranulin could result in frontotemporal lobar degeneration through the phosphorylation of the specific site of the tau protein.

In addition, the present inventors examined the relation between the progranulin gene mutation and the abnormal phosphorylation of Ser203 in the tau protein. Consequently, progranulin was first found to inhibit the binding of Tyro3, a receptor tyrosine kinase, with Gas6, a secretory protein. On the other hand, when the expression of progranulin is reduced, Gas6 binds to Tyro3 to activate Tyro3. It has been found that signals downstream of Tyro3, i.e., a predetermined kinase (PKCs, MAPK, BRAF, etc.) is consequently activated, ultimately resulting in the abnormal phosphorylation of Ser203 in the tau protein.

Then, activation of these kinases was suppressed in the PGRN-KI mice using an inhibitor or an AAV-knockdown vector for a predetermined kinase (PKCα, which is one of the PKCs, MAPK, or BRAF). This was found to normalize the number of synapses that had been reduced, and ameliorated the symptoms of the cognitive impairment.

In light of the results described above, it has been found that frontotemporal lobar degeneration (FTLD-TD, in particular) can develop mainly according to a mechanism that proceeds in the following order:
(1) reduced expression of progranulin;
(2) binding of Gas6 with Tyro3 and resultant activation of Tyro3;
(3) activation of signals downstream of Tyro3 (specifically, the activation of a predetermined kinase (PKCs, MAPK, BRAF, etc.);
(4) phosphorylation of the specific site (Ser203 for mice) in the tau protein;
(5) intracerebral aggregation of the TDP43 protein and the tau protein; and
(6) degeneration of synapses and development of frontotemporal lobar degeneration.

Thus, a novel finding has been found that an active ingredient capable of acting on the above mechanism and consequently suppressing the phosphorylation at the specific site in the tau protein (Ser203 for mice) could be a therapeutic agent for frontotemporal lobar degeneration.

### (Active Ingredient)

The therapeutic agent of the present invention contains an active ingredient (hereinafter, also referred to as "active ingredient in the present invention") which suppresses the gene expression or protein function of Gas6 and/or Tyro3. In other words, the active ingredient in the present invention has an effect of suppressing the binding of Gas6 with Tyro3 and an effect of suppressing the activation of Tyro3 caused by the binding of Gas6 with Tyro3. Thus, according to the therapeutic agent of the present invention, since the binding of Gas6 with Tyro3 and the signals downstream of the resultant activation of Tyro3 are suppressed, the effect of suppressing the phosphorylation at the specific site in the tau protein (Ser203 for mice), as well as the effect of treating the degeneration of the synapses and the development of the frontotemporal lobar degeneration, for example, are exhibited.

In the present invention, "suppressing gene expression" and related phrases mean that the gene is not expressed, or that the expression of the gene is reduced as compared with a case where the active ingredient in the present invention is not administered.

In the present invention, "suppressing protein function" and related phrases mean that the protein does not function, or that the functional expression of the protein is reduced as compared with a case where the active ingredient in the present invention is not administered.

Although there is no particular limitation on embodiments of the active ingredient in the present invention, those containing a nucleic acid, for example, are preferred, and those composed of a nucleic acid are preferred. When the active ingredient in the present invention contains the nucleic acid, the nucleic acid is usually prepared as one capable of suppressing the expression of the gene of Gas6 and/or Tyro3, or capable of knocking down the gene. The active ingredient in the present invention may be used either alone as one type, or in a combination of two or more types thereof.

The nucleic acid may be an shRNA, an siRNA, a dsRNA, an miRNA, an antisense nucleic acid, or the like. The active ingredient in the present invention is preferably an shRNA of the gene of Gas6 and/or Tyro3.

Embodiments of the active ingredient in the present invention may include antibodies (polyclonal antibodies, monoclonal antibodies, functional fragments of antibodies, and the like) or small compounds.

The amount of the active ingredient in the present invention in the therapeutic agent of the present invention can be appropriately adjusted depending on the effects to be obtained, the weight and symptoms of the patient, and the like.

The therapeutic agent of the present invention may contain an active ingredient other than the aforementioned active ingredient in the present invention, without inhibiting the action of the active ingredient in the present invention. However, it is preferred, from the viewpoint of easily exhibiting the effects of the present invention, that the therapeutic agent of the present invention contains the active ingredient in the present invention alone as an active ingredient.

### (Other Ingredients)

Other pharmacologically acceptable ingredients may be blended into the therapeutic agent of the present invention, without inhibiting the action of the active ingredient in the present invention or other active ingredient(s) optionally contained. Such other ingredients are exemplified by a carrier, an excipient, a disintegrant, a buffer, an emulsifier, a suspending agent, a stabilizer, a preservative, an antiseptic agent, and physiological saline. As the excipient, lactose, starch, sorbitol, D-mannitol, white sugar, or the like may be used. As the disintegrant, starch, carboxymethylcellulose, calcium carbonate, or the like may be used. As the buffer, phosphates, citrates, acetates, or the like may be used. As the emulsifier, gum arabic, sodium alginate, tragacanth, or the like may be used. As the suspending agent, glycerol monostearate, aluminum monostearate, methylcellulose, carboxymethylcellulose, hydroxymethylcellulose, sodium lauryl sulfate, or the like may be used. As the stabilizer, propylene glycol, diethylin sulfite, ascorbic acid, or the like may be used. As the preservative, phenol, benzalkonium chloride, benzyl alcohol, chlorobutanol, methylparaben, or the like may be used. As the antiseptic agent, sodium azide, benzalkonium chloride, paraoxybenzoic acid, chlorobutanol, or the like may be used. The type and amount of these ingredients may be appropriately adjusted depending on the effects to be obtained, and the like.

### (Dosage Form)

Dosage forms of the therapeutic agent of the present invention are not particularly limited as long as they can be used as a medicine. Examples of the dosage forms include oral formulations (tablets and the like), parenteral formulations (injectables and the like), and the like. These dosage forms can be prepared according to conventional processes in the pharmaceutical field.

When the dosage form of the therapeutic agent of the present invention is a parenteral formulation, intravenous administration, intraarterial administration, intraperitoneal administration, subcutaneous administration, intradermal administration, tracheobronchial administration, rectal administration and intramuscular administration, administration by an infusion, direct administration to a target site (brain or the like), and the like may be performed depending on the type of the dosage form. The direct administration to the target site is preferred in light of greater therapeutic effects and a reduced amount of the agent to be administered. The administration to the target site may be performed using, for example, a cannula (catheter), an incision, a drug delivery system, an injection, or the like. More specifically, a method in which a cannula or the like is inserted by a stereotaxic operation procedure and the agent is administered into the brain through the cannula, a method in which the head is opened and a sustained release drug delivery system (e.g., an osmotic pump manufactured by ALZET Corporation) containing the agent is embedded into the brain, and a method in which the agent is introduced into cells in the brain by electroporation can be mentioned. In addition, when the agent of the present invention is not directly administered into the brain, a method in which the compound is conjugated with a brain barrier permeable substance, and then the conjugate is administered may be utilized. It is to be noted that the brain barrier permeable substance is exemplified by, but not limited to, a glycoprotein composed of 29 amino acids derived from rabies virus (see, Nature, vol. 448, pp. 39-43, July 5, 2007).

The number of administrations of the therapeutic agent of the present invention can be appropriately adjusted according to the dosage of the active ingredient in the present invention, the route of administration, and the like. <Screening Method for Therapeutic Agent for Frontotemporal Lobar Degeneration>

A method for screening a therapeutic agent for frontotemporal lobar degeneration according to an embodiment of the present invention (hereinafter, also referred to as "screening method of the present invention") includes administering a candidate compound to a disease model mouse of frontotemporal lobar degeneration, in which the progranulin gene of the disease model mouse is modified by knock-in, and screening the candidate compound based on the phosphorylation of Ser203 in tau protein as an indicator.

In disease model mice of frontotemporal lobar degeneration in which the progranulin gene of the disease model mice was modified by knock-in, the expression of progranulin is decreased. Therefore, in the disease model mice, the mechanism explained above proceeds and the degeneration of the synapses and the development of frontotemporal lobar degeneration are found. If the candidate compound is administered to such disease model mice and the suppression of the phosphorylation of Ser203 is found in the disease model mice as compared with disease model mice to which the candidate compound is not administered, the candidate compound can be expected to function as a therapeutic agent for frontotemporal lobar degeneration. In the present invention, "phosphorylation of Ser203 is suppressed as compared with disease model mice to which a candidate compound is not administered" and related phrases mean that the amount of the phosphorylation of Ser203 is reduced as compared with the disease model mice to which the candidate compound is not administered, or that the phosphorylation of Ser203 is not found.

Although the method for knocking in the progranulin gene is not particularly limited, the method shown in the Examples or the method described in WO2015/099094 can be employed. Although the type and sex of the mice are not particularly limited, C57BL/6J male mice are preferred because of ease of handling and availability.

The candidate compound is not particularly limited, and examples thereof include expression products of gene libraries, synthetic small compound libraries, peptide libraries, antibodies, substances released by bacteria, extracts and culture supernatants of cells (microorganisms, plant cells, or animal cells), purified or partially purified polypeptides, extracts from marine organisms, plants or animals, soils, and random phage peptide display libraries.

The presence or absence, or amount of the phosphorylation of Ser203 can be identified by performing detection (Western blotting, etc.) using an anti-phosphorylated Ser203 antibody on a tissue (e.g., cerebral tissue) of the disease model mice. Specific methods may include, for example, the method described in Fujita et al. (Nat Commun. 2018 Jan 30; 9(1): 433; doi: 10.1038/s41467-018-02821-z).

In the screening method of the present invention, the criterion for the candidate compound to function as a therapeutic agent for frontotemporal lobar degeneration may be only that the phosphorylation of Ser203 is suppressed as compared with the disease model mice to which the candidate compound is not administered. In addition, it may be further added to the criteria that the suppression of the degeneration of the synapses and/or the suppression of the symptoms of frontotemporal lobar degeneration can be found as compared with the disease model mice to which the candidate compound is not administered. The presence or absence, or degree of the degeneration of the synapses can be determined by performing the morphological analysis of spines according to the method described in the Examples. The presence or absence, or degree of the symptoms of frontotemporal lobar degeneration can be determined by performing behavioral experiments according to the method described in the Examples.

### <Treatment Method for Frontotemporal Lobar Degeneration>

A method for treating frontotemporal lobar degeneration according to an embodiment of the present invention (hereinafter, also referred to as "treatment method of the present invention") includes administering a therapeutic agent of the present invention to a patient.

The dosage, the number of administrations, the interval of administrations, the route of administration, and the like of the therapeutic agent of the present invention can be appropriately adjusted according to the effects to be obtained, the weight and symptoms of the patient, and the like.

A subject for administration of the therapeutic agent of the present invention is any subject that is developing a symptom of frontotemporal lobar degeneration. The type of the subject for administration is not particularly limited, and examples thereof include humans and mammals other than humans (mice, rats, dogs, cats, pigs, cows, and the like).

### EXAMPLES

Hereinafter, the present invention will be described in more detail by way of Examples, but the present invention is not limited to these Examples in any way.

### <Preparation of Disease Model of Frontotemporal Lobar Degeneration>

Knock-in of progranulin was performed using C57BL/6J male mice in the following manner to prepare disease model mice of frontotemporal lobar degeneration. The resulting mice had a R504X mutation. Hereinafter, the progranulin knock-in mice are also referred to as "PGRN-KI mice".

A heterozygous PGRN R504X mutation was constructed by introducing a Neo-cassette (manufactured by Unitech Japan) into C57BL/6J mice. It is to be noted the Neo-cassette present in F1 mice was removed by mating with CAC-Cre mice.

A targeting vector was prepared from the following two constructs. A BamHI (Blunt)-XhoI fragment obtained from construct 2 was subcloned between XhoI (Blunt) and SalI sites of construct 1. The resulting plasmid was used as a targeting vector.

### (Construct 1)

A PCR product of a 6.5 kbp NotI-XhoI fragment (including R504X mutation) amplified from a BAC clone (ID: RP23-311P1 or RP23-137J17) was subcloned into a vector pBS-DTA (manufactured by Unitech Japan).

### (Construct 2)

A PCR product of a 3.0 kbp ClaI-XhoI fragment was subcloned into a vector pBS-LNL(-) containing the Neo-cassette.

After the targeting vector was linearized with SwaI, the resulting fragment was introduced into ES-clones of the C57BL/6J mice by electroporation. Genotyping of the ES clones was performed by PCR using the following primers:
5'-CGTGCAATCCATCTTGTTCAAT-3' (forward)
5'-CATGACCTAACTCAATGCATACCAC-3' (reverse)

Positive clones were subjected to Southern blotting analysis.

5' and 3' probes for Southern blotting were prepared as follows:
5'-Probe
5'-CTGTGTCTCACTAGAAGCATAAGCA-3' (forward)
5'-ACTAGATTGGGAAAGACAGTGAATC-3' (reverse)
3'-Probe
5'-AATTCCAATCCTGTGTGGTCATAG-3' (forward)
5'-CCACTTTCTTTCTCCCTTACCCTA-3' (reverse)

A neomycin probe was prepared with the following primers:
5'-GAACAAGATGGATTGCACGCAGGTTCTCCG-3' (forward)
5'-GTAGCCAACGCTATGTCCTGATAG-3' (reverse)

The presence of the introduced progranulin mutation was confirmed by PCR using a genomic DNA prepared from tail snips. Amplification was performed with "KOD-FX neo" (manufactured by Toyobo Co., Ltd.) using the following primers:
5'-GATCTTTCTATGTACAGCCACGTTT-3' (forward)
5'-CTAATGGTGTTCAACGTCAAGTAGT-3' (reverse)
The PCR conditions are as follows:
98°C for 10 s (denaturation), 57°C for 30 s (annealing), and
68°C for 45 s (extension) for 35 cycles.

The amplicon from the genome of the mice without the knock-in contained only a 302 bp fragment, whereas the amplicon from the genome of the mice with the knock-in contained a 302 bp fragment and a 392 bp fragment.

The obtained PGRN-KI mice were evaluated from the following viewpoints, and it was confirmed that these mice developed the disease state of frontotemporal lobar degeneration.

### (Investigation of Localization of Aggregated Proteins of TDP43)

Among several types of frontotemporal lobar degeneration, the one which is classified as "FTLD-TDP" is known to have an aggregated protein of TDP43 localized in the cytoplasm of the neurons in the frontal and temporal lobes. In normal neurons, TDP43 is localized in the nucleus. Thus, the localization of the aggregated protein of TDP43 in the neurons of the frontal lobe of the PGRN-KI mice was investigated using an anti-TDP43 antibody and an anti-phosphorylated TDP43 antibody, and it was confirmed that the aggregated protein was localized in the cytoplasm, not in the nucleus, of the neurons.

### (Behavioral Experiments with PGRN-KI Mice)

The following two types of behavioral experiments (fear conditioning experiment and Morris water maze test) were performed, and an impaired cognitive response was found in the PGRN-KI mice of 12 weeks or older. Such an impaired response is a symptom similarly seen in frontotemporal lobar degeneration. It is to be noted that all behavioral tests are carried out according to the method indicated by Ito H. et al. (Mol Psychiatry. 2015 Apr; 20 (4): 459-471).

### [Behavioral Experiment; Fear Conditioning Experiment]

A fear conditioning experiment was performed with the PGRN-KI mice and control mice (C57BL/6J mice without the knock-in). Specifically, the mice received electrical stimulation (0.4 mA, 2 s) on their paws along with sound stimulation (65 dB white noise, 30 s). Twenty-four hours later, the frequency of the freezing reaction in the mice was measured when the mice were subjected to the sound stimulation without the electrical stimulation in the same chamber. As a result, the PGRN-KI mice were found to have significantly shorter time periods of the freezing behavior as compared with the control mice. This means that the cognitive response of the PGRN-KI mice was impaired as compared with the control mice.

### [Behavioral Experiment; Morris Water Maze Test]

A Morris water maze test was performed with the PGRN-KI mice and control mice (C57BL/6J mice without the knock-in). Specifically, the mice received a 60-second trial four times per day for 5 days, and the time period taken for each mouse to reach the platform was measured. As a result, the PGRN-KI mice were found to have significantly shorter residence time periods at the target area as compared with the control mice. This means that the cognitive response of the PGRN-KI mice was impaired as compared with the control mice.

### <Comprehensive Phosphoproteomic Analysis Using Disease Model of Frontotemporal Lobar Degeneration>

A comprehensive phosphoproteomic analysis was performed over time using cerebral tissues from the PGRN-KI mice, and it was found that frontotemporal lobar degeneration can develop mainly by a mechanism that proceeds in the following order: A schematic diagram of the mechanism is shown in FIG. 1.
(1) reduced expression of progranulin;
(2) binding of Gas6 with Tyro3 and resultant activation of Tyro3;
(3) activation of signals downstream of Tyro3 (specifically, the activation of a predetermined kinase (PKCs, MAPK, BRAF, etc.);
(4) phosphorylation of the specific site (Ser203 for mice) in the tau protein;
(5) intracerebral aggregation of the TDP43 protein and the tau protein; and
(6) degeneration of synapses and development of frontotemporal lobar degeneration.

### <Investigation of Therapeutic Effects Produced by Knockdown of Various Genes>

In accordance with the findings obtained in the aforementioned section "Comprehensive Phosphoproteomic Analysis Using Disease Model of Frontotemporal Lobar Degeneration", it can be expected that inhibition of any step of the aforementioned mechanism and consequent suppression of the phosphorylation at the specific site (Ser203 for mice) in the tau protein would enable the treatment of frontotemporal lobar degeneration. Thus, various genes (tau, BRAF (also referred to as b-raf), PKCα, Tyro3, and Gas6) that could be involved in the development of frontotemporal lobar degeneration were knocked down in the PGRN-KI mice and control mice (C57BL/6J mice without the knock-in) using an shRNA, and a behavioral experiment was performed in the same manner as the section "Preparation of Disease Model of Frontotemporal Lobar Degeneration" described above, and morphological analysis of spines (postsynaptic regions) was further performed.

The knockdown of each gene was performed according to the following procedures: Lentiviral particles (2 × 10⁵ titer units/100 µl) containing scrambled shRNA (sc-108080, manufactured by Santa Cruz Biotechnology), Tau-shRNA (sc-430402-V, manufactured by Santa Cruz Biotechnology), B-Raf shRNA (sc-63294-V, manufactured by Santa Cruz Biotechnology), or Tyro3-siRNA (iV037848, manufactured by ABM) were diluted to 1:4 with ACSF buffer (NaCl 125 mM; KCl 2.5 mM; NaH₂PO₄ 1.25 mM; MgCl₂ 1 mM; CaCl₂ 1 mM; NaHCO₃ 26 mM; and glucose 25 mM) . Further, a plasmid vector (10 µg) containing scrambled shRNA (TG501653, manufactured by OriGene), PKCα-shRNA (TG501653, manufactured by OriGene), or Gas6-shRNA (sc-35451-SH, manufactured by Santa Cruz Biotechnology) was dissolved in 100 µl of in vivo-jetPEI (201-10G, manufactured by Polyplus-transfection). Each shRNA was administered with an osmotic pump (0.15 µl/h) into the subarachnoid space of the PGRN-KI mice and the control mice (C57BL/6J mice without the knock-in) over the period from 8 to 12 weeks of age of the mice.

### (Behavioral Experiment)

The PGRN-KI mice knocked down for each gene (tau, BRAF, PKCα, Tyro3, or Gas6) exhibited a significantly improved cognitive response in the results of both the fear conditioning experiment and the Morris water maze test as compared with the unknocked-down PGRN-KI mice.

### (Morphological Analysis of Spines)

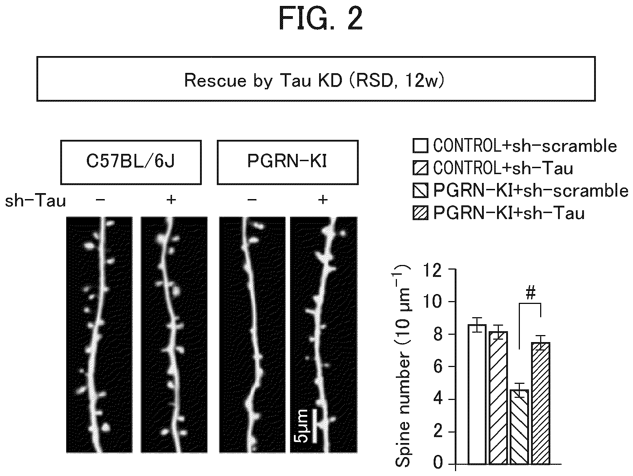

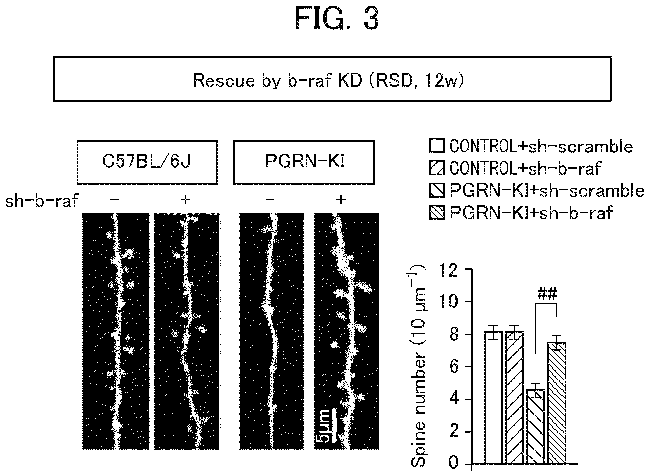

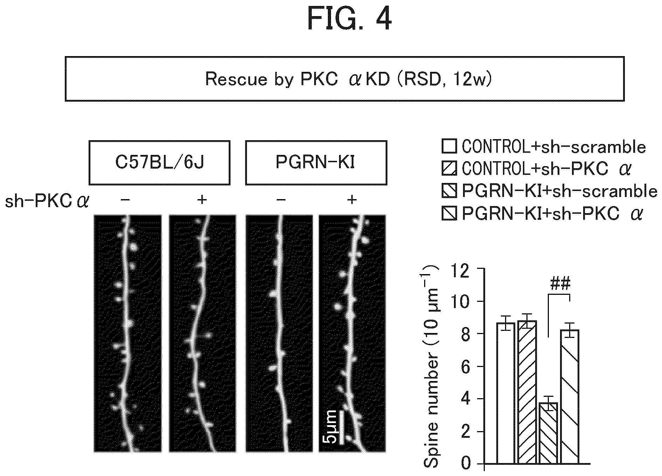

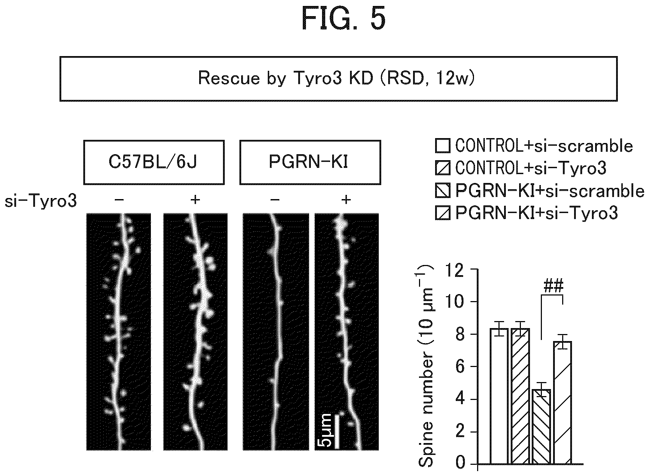

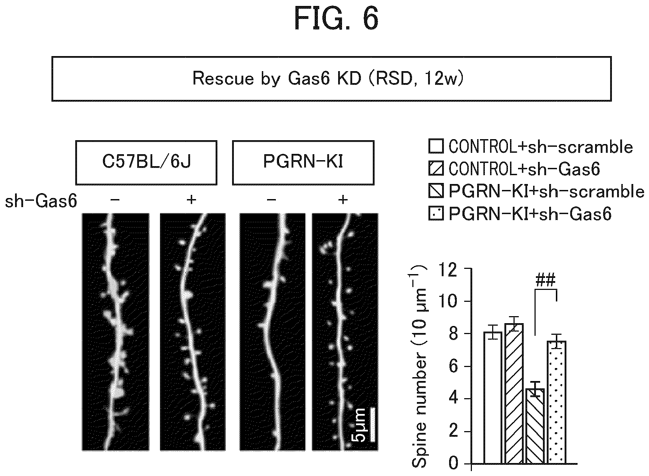

Images of dendrites from the mice (at 12 weeks of age) knocked down for each gene were acquired using a two-photon excitation microscope. In addition, the number of spines was measured using "IMARIS software" (IMARIS 7.2.2, manufactured by Bitplane GmbH). The results from the knockdown of tau are shown in FIG. 2, the results from the knockdown of BRAF are shown in FIG. 3, the results from the knockdown of PKCα are shown in FIG. 4, the results from the knockdown of Tyro3 are shown in FIG. 5, and the results from the knockdown of Gas6 are shown in FIG. 6.

As shown in FIGs. 2-6, the PGRN-KI mice knocked down for each gene (tau, BRAF, PKCα, Tyro3, or Gas6) had a markedly higher number of spines as compared with the unknocked-down PGRN-KI mice, and were comparable to the control mice. On the other hand, no change in the number of spines was found in the control mice with or without the knockdown. This means that the knockdown of each gene (tau, BRAF, PKCα, Tyro3, or Gas6) normalized the degeneration of the synapses in the PGRN-KI mice.

### <Reference; Investigation of Therapeutic Effect of Frontotemporal Lobar Degeneration Using Various Inhibitors>

According to the findings obtained in the aforementioned section <Comprehensive Phosphoproteomic Analysis Using Disease Model of Frontotemporal Lobar Degeneration>, it can be expected that inhibition of any step of the aforementioned mechanism and consequent suppression of the phosphorylation at the specific site (Ser203 for mice) in the tau protein would enable the treatment of frontotemporal lobar degeneration. Thus, vemurafenib (BRAF inhibitor) or Go6976 (PKC inhibitor) was administered to the PGRN-KI mice, and a behavioral experiment and morphological analysis of spines were performed in the same manner as described in the aforementioned section

### <Investigation of Therapeutic Effects Produced by Knockdown of Various Genes>.

### (Vemurafenib-Administered Group)

Vemurafenib (32 mg/kg of body weight/day) was orally administered to the PGRN-KI mice and control mice (C57BL/6J mice without the knock-in) over the period from 6 to 12 weeks of age of the mice. A group of mice that received a mock instead of vemurafenib served as a control group.

### (Go6976-Administered Group)

Into the subarachnoid space of the PGRN-KI mice and control mice (C57BL/6J mice without the knock-in) was administered 4.4 µM Go6976 (manufactured by Calbiochem) with an osmotic pump (0.15 µl/h, #2006, manufactured by DURECT Corporation) over the period from 10 to 12 weeks of age of the mice. A group of mice that received PBS instead of Go6976 served as a control group.

### (Behavioral Experiments)

The PGRN-KI mice to which vemurafenib or Go6976 was administered showed a significantly improved cognitive response in the results of both the fear conditioning experiment and the Morris water maze test as compared with the PGRN-KI mice to which neither vemurafenib nor Go6976 was administered. On the other hand, no change in the cognitive response was found in the control mice with or without vemurafenib or Go6976.

### (Morphological Analysis of Spines)

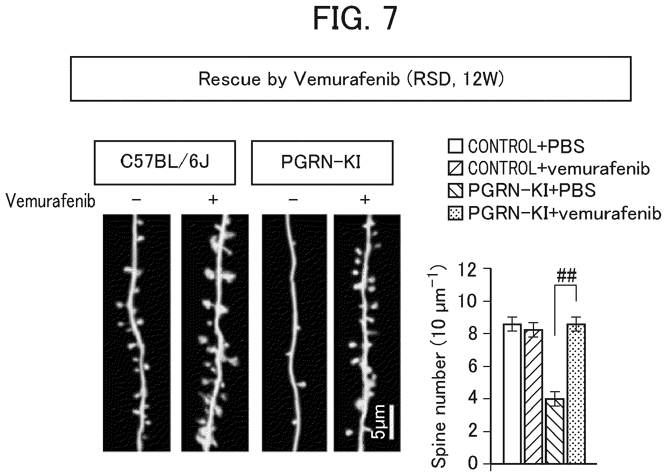

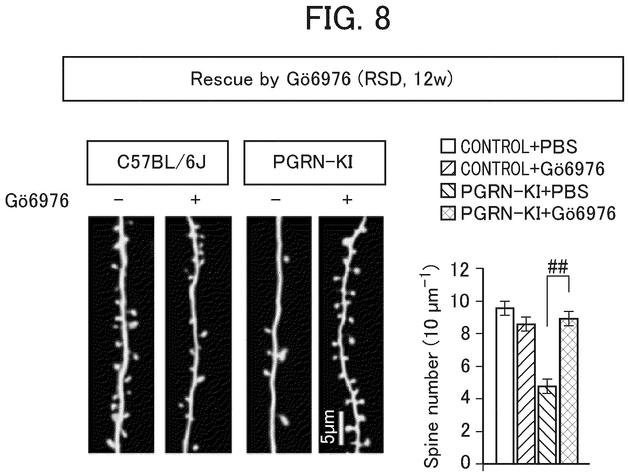

Images of dendrites from the mice (at 12 weeks of age) in each administered group were acquired using a two-photon excitation microscope. In addition, the number of spines was measured using "IMARIS software"(IMARIS 7.2.2, manufactured by Bitplane GmbH). The results of the vemurafenib-administered group are shown in FIG. 7, and the results of Go6976-administered group are shown in FIG. 8.

As shown in FIGs. 7 and 8, the PGRN-KI mice receiving vemurafenib or Go6976 had a markedly higher number of spines as compared with the PGRN-KI mice not receiving vemurafenib or Go6976, and were comparable to the control mice. On the other hand, no change in the number of spines was found in the control mice with or without vemurafenib or Go6976. This means that the administration of vemurafenib or Go6976 normalized the degeneration of the synapses in the PGRN-KI mice.

## Claims

1. A therapeutic agent for frontotemporal lobar degeneration, comprising an active ingredient which suppresses gene expression or protein function of Gas6 and/or Tyro3.

2. The therapeutic agent for frontotemporal lobar degeneration according to claim 1, wherein the active ingredient comprises a nucleic acid.

3. A method for screening a therapeutic agent for frontotemporal lobar degeneration, comprising administering a candidate compound to a disease model mouse of frontotemporal lobar degeneration, wherein a progranulin gene of the disease model mouse is modified by knock-in, and screening the candidate compound based on phosphorylation of Ser203 in tau protein as an indicator.

4. A method for treating frontotemporal lobar degeneration, comprising administering to a patient the therapeutic agent for frontotemporal lobar degeneration according to claim 1 or 2.
